# EUROPEAN PATENT SPECIFICATION

(11) **EP 2 594 919 B1**
(45) Date of publication and mention of the grant of the patent: **27.11.2019**
(21) Application number: 11867572.7
(22) Date of filing: 26.08.2011
(51) Int. Cl.: G01N 21/55, G01N 21/17, G01N 21/86, G01N 21/84, G01N 33/22, G01N 33/48, A61B 5/145, G01N 21/27, G01N 33/18, G01N 33/557

(54) **ASSAY DETECTION JUDGMENT APPARATUS AND METHOD**
VORRICHTUNG UND VERFAHREN ZUR BEURTEILUNG VON TESTERGEBNISSEN
APPAREIL ET PROCÉDÉ DE JUGEMENT DE DÉTECTION D'ANALYSE

(30) Priority: 05.08.2011 CN 201110223436
(43) Date of publication of application: 22.05.2013
(73) Proprietor: Guangzhou Wondfo Biotech. Co., Ltd., Guangdong 510663 (CN)
(72) Inventor: WANG, Jihua, Guangzhou, Guangdong 510663 (CN); LUO, Shiyao, Guangzhou, Guangdong 510663 (CN)
(74) Representative: Potter Clarkson
(86) International application number: PCT/CN2011/078970
(87) International publication number: WO 2013/020308

(56) References cited:
- WO-A1-2009/144507
- CN-A- 101 639 451
- CN-A- 102 105 795
- GB-A- 2 402 475
- US-A- 4 523 279
- US-A- 4 553 838
- US-A1- 2006 008 896
- US-A1- 2009 061 534

## Description

### FIELD OF THE INVENTION

The present invention relates to a method and device for determining assay results, especially to a method and device for assay determination by reading the measured value of test objects.

### BACKGROUND OF THE INVENTION

In the market, there are many devices for analyte measurement. For example, a type of cross flow immunoassay device, which is used to measure HCG, is a disposable test strip. This device needs the user to read the result, which has a certain degree of subjectivity and requires an extra timer. The result exhibits in color, which will get deeper with time increasing and affect the determination. A further known device utilizes the optical method to obtain results, which inserts a test strip into a reading device, positions the test strip on the optical element of the reading device and exposes it under the light beam emitted from light source, and the reflection and transmission lights will be checked by a light detector. Typically, the reading device which is an electronic pen used for multiple times, includes at least one light-emitting diode, and each light-emitting diode is set with a corresponding light detector. These similar analyzing devices, need carefully position reading device and test strip for analysis, since the produced visible signal is quite weak in the detection and control zone and even tiny misplacement in corresponding detection and control zone significantly affects reading of the detection device. In addition, it is important for light detector being close to the test strip because the light amount captured by photosensitive diode is very small. The signal intensity follows an inverse-square law, and therefore, it dramatically decreases by the increasing distance between the test strip and light detector. Thus, it is necessary for the user to carefully position the test stick and the reading device for result analyzing, especially for household devices. The analyzing device which is used for multiple times, especially the small size one, is easily caused by repeated insertion, while a tiny dislocation will in turn cause an inaccuracy.

A further method for automatic immunity device, detects if there is fluid on the carrier via additional electrode provided by the cross flow carrier, and generates a signal to connect the electronic detection device, displays the detected result. But in different tests, fluids migrate at different speeds along the carrier and different characteristics of flow velocity of liquids will cause inaccurate result. For the inconsistent material natures of imbibition core and porous water-permeable membrane, the optimal timings for reading result are different. A corresponding device for reading test result is used to read the test result by utilizing execution of fluid transportation carrier. It solves the problem about how to determine the optimal reading timing, but can not solve the problem about different flow velocity standards of different fluids. Meanwhile, testing time of some testing and analyzing devices is too long, e.g. the business cross flow detection device for detecting heart damage on the basis of laboratory test which requires 15 min to finish the test. In other situations, especially in a pregnancy test, the user wants to know the result as soon as possible for sure.

There is also a calculation method, which sets two threshold values, and obtains detecting result by comparing the relation between measured value and threshold value. But since the designed reaction needs a certain time to reach equilibrium for detection, the fast detecting results will cause the instability of the results. Meanwhile, the invention does not mention whether the threshold value is changeable. Based on research, according to the different lasting time, the dry and wet degree of the test strip will change, and threshold value may change, but the fixed threshold value will cause certain error. The detecting results by only measuring the accumulated amount of signals in the detection zone and lacking the contrast of blank zone, will have bigger error without eliminating influencing factors in background. In addition, inappropriate operation will happen easily in the replacement of test strips, which will cause determining error due to a little error by reading device which is sensitive to the optical length.

US2009/061534 describes an assay device for determining the presence and/or extent of one or more analytes in liquid sample containing a) first and second assays each comprising a flow-path having a detection zone for immobilising a labelled binding reagent, wherein detection of a labelled binding reagent at one or both detection zones is indicative of the presence and/or extent of one or more analytes; b) a shared reference zone; c) one or more light sources to illuminate the detection zones and the reference zone; d) one or more photodetectors to detect light from the detection zones and the reference zone, which photodetector/s generate a signal, the magnitude of which signal is related to the amount of light detected; and e) signal processing means for processing signals from the photodetector/s. Document WO-A-2009/144507 further discloses an assay device for determining the presence or extent of an analyte.

### SUMMARY OF THE INVENTION

It is an object of the present invention to provide a method and device for determining assay results, which work more efficiently, more accurately and cost less.

According to an aspect of the invention, there is provided a device as recited in Claim 1. According to another aspect of the invention, there is provided a method as recited in Claim 7. Optional features of the invention are set out in the dependent claims.

To achieve the object of the present invention, the following technical solutions are provided:
A device for assay determination, according to claim 1 comprises:
a photoelectric detection circuit;
a processor; and
an optical detection device for measuring assay signals, provided with a detection zone and a blank zone, wherein the photoelectric detection circuit detects light-reflection intensity signals from the detection zone and the blank zone, and feeds back detected information to the processor;
wherein the processor comprises a time controlling module, a threshold modification module and a datum comparison module which are connected to each other, the time controlling module is connected with the threshold modification module and the photoelectric detection circuit separately, and the threshold modification module and the photoelectric detection circuit are connected with the datum comparison module at the same time, the threshold modification module modifies threshold value according to signals from the time controlling module which is then sent to the datum comparison module, the photoelectric detection circuit measures obtained detection value according to the signal of time controlling module which is then sent to the datum comparison module, and the datum comparison module obtains the determined result by calculating and comparing detection data with the corresponding threshold value.

Preferably, the threshold modification module comprises a starting-up testing module, a time measuring module, a threshold updating module and a threshold output module which are connected with each other. The threshold modification module detects the starting state (i.e. the circuit is switched on) according to the starting-up test module, the time test module detects the time signal from timing control module, and based on the time signal, the threshold value update module updates the new threshold value according to a preset relation of the threshold value variation with time, and output the updated threshold value to the datum comparison module through the threshold output module.

The datum comparison module comprises a blank value protection module, a measured value input and a protection module, a gradient calculation module, a comparison and determination module, and a result saving module. Values of the blank value protection module, the measured value input and protection module are inputted into a gradient calculation module, and the result of the gradient calculation module is inputted into the comparison and determination module and compared with the threshold value. The determined result is stored into the result saving module.

Preferably, the device for determining the assay results further comprises a displaying device for displaying the measured status or determined result, and the displaying device is connected with the processor. Preferably, the displaying device is LCD liquid crystal screen. As a preferred embodiment, the LCD liquid crystal screen shows the detection status with an hourglass lightning picture.

Preferably, the device for determining the assay result further comprises voice alarming device connected with the processor. The determined result or detection status can be presented by sound.

The device is used for reading result of laboratory test to measure the existence and content of a target analyte, wherein the existence or non-existence of the target analyte gives rise to a reaction and cause a signal accumulation with the time changing. The device is used to determine the accumulated signal cumulant, and calculate the determined value and compare it with the threshold value, and display the result of laboratory test. Therefore, the device can guarantee the fast speed of obtaining the result and meanwhile the accurate result of test. It avoids the disadvantages that error happens within a short time after adding sample or that the waiting time is too long. Meanwhile, the most accurate result of the test is obtained at the fastest speed by fully considering the water loss of test strip and setting a relocatable threshold value. The set of blank zone can remove the background, and reduce errors of measuring.

A method for determining the assay result according to claim 7 by using the device as mentioned above. The method is used to measure the existence and content of the analyte to be determined, perform the laboratory test to give rise to a reaction; the reaction causes a laboratory test signal which accumulates with time in detection zone of the optical detection device, the photoelectric detection circuit emits light to detection zone and the blank zone, and measures the light-reflection intensity signal of the detection zone and the blank zone, and feeds back detected information to the processor, wherein,
the processor is preset with a threshold value X changing with time;
the processor defines accumulated amount of the signal of laboratory test and calculates corresponding determined value K, according to the light-reflection intensity signals of both before and after adding sample of the detection zone and the blank zone, which are fed back by photoelectric detection circuit.

When the confirmed determined value K is bigger than or equal to the current threshold value X, or at the time t, reaches the preset time value, the determined value K doesn't not reach the threshold value, or detected signal can not be determined, display the result.

Preferably, if within a certain time, after the reaction, the determined value K is bigger than or equal to the preset threshold value X (i.e. K≥X), then immediately output the first result signal, and end the assay; if the time t reaches the preset value t4, and the determined value K is smaller than preset threshold X (i.e. K<X), output the second signal and end the assay; if non-comparison status appears, output the third result and end the assay. The non-comparison status usually happens when no sample is added. While t4 is determined according to the actual situation, accurate results will be obtained by knowing the time of t4 from laboratory data.

Preferably, within the preset time t4, if the determined value is lower than the preset threshold value, then repeat a new round of the measuring, calculating and comparing.

Preferably, the first result is a positive result, the second result is a negative result, and the third result is a result of unable to judge or insufficient sample.

Preferably, the processor determines accumulation amount of assay signal before the reaction reaches equilibrium. Because the reaction of assay takes a long time, it also takes a long time to read the data after the reaction is finished, and the sampling amount of the reaction process is linearly proportional to time, thus accurate data of assay will be obtained by quantitative detection to assay signals based on proportional relation between the sampling amount and the time. In addition, when the reaction reaches equilibrium, the wetness of the detection zone is decreasing with time passing by, the color of the detection zone gets gradually deeper, but the assay signal of the reaction does not increase, so the detected signal has a great possibility that it is not consistent with the real result of the reaction, but in the reaction process, the color change of the detection zone is proportional to the reaction amount, and the detected signal in this stage can truly reflect the exact result of the chemical reaction.

Preferably, the determined value K is determined by the following: scan and read values of T0 and B0 before sampling in the detection zone and the blank zone, and compare individually with the values of T1 and B1 which are scanned and read in the detection zone and the blank zone, and obtain the ratio of T1/T0 in the detection zone and ratio of B1/B0 in the blank zone, and the determined value K=T1/T0-B1/B0 which is obtained by subtracting the ratio of blank zone from the ratio of detection zone. The N second can be chosen by relation between reaction amount of a specific reaction of assay and time.

Preferably, the detection operation is following the preset time.

Preferably, after adding sample, when time t reaches t1, a time alarming sign appears, preferably utilizing sound as alarm to indicate that the sampling reaction starts. Wherein, t1 is an estimated reaction time after adding sample to the detection zone.

Preferably, after adding sample, when the time t reaches t2, read values of T and B of the detection zone and blank zone, if no sample is added, which is T=T0 and B=B0, output the third result, and the third result is outputted in a way of error alarm, specifically in a outputting way of displaying, and/or sound.

Preferably, after t3 interval time from the above T2 time when reading values of T and B, read the values of T1 and B1 of the detection zone and blank zone, and calculate determined value K based on values of T1 and B1, and compare K with the threshold value X. Within preset time t4, if the determined value K is lower than preset threshold value X, then repeat a new round of operation, i.e. every t3 time interval, measured values of T1 and B1, and calculate determined value K and compare it with the threshold value X.

Compared with the prior art, the present invention has advantages as following: the device and method for determining the result of assay work more efficiently, measure more accurately and cost less than the prior art. The present invention can guarantee the fast speed of obtaining the result and meanwhile the accurate result of test. It avoids the disadvantage that display error happens within a short time after adding sample or that the waiting time is too long. Meanwhile, the most accurate result of the test is obtained at the fastest speed by fully considering the water loss of test strip and setting a relocatable threshold value.

### BRIEF DESCRIPTION OF THE DRAWINGS

Fig. 1 is a frame structural view of the device for determining the result of assay.
Fig. 2 is a schematic view of the threshold modification module of the device for determining the result of assay.
Fig. 3 is a schematic view of the datum comparison module of the device for determining the result of assay.
Fig. 4 is a flow chart of an embodiment of the method for determining the result of assay.

### DETAILED DESCRIPTION OF THE PREFERRED EMBODIMENTS

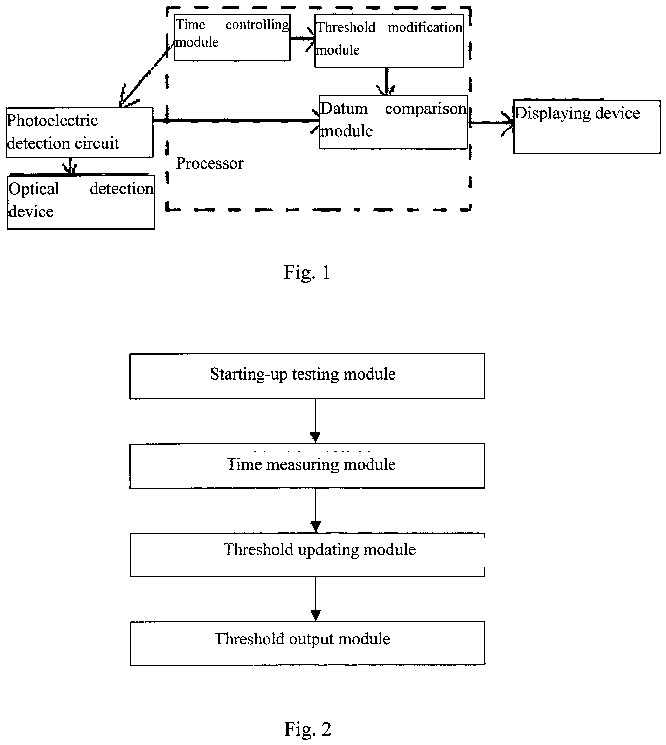

Referring to Figs. 1 to3, a device for determining the result of assay in the present invention accompanying with the figures is shown. The device for determining the result of assay includes a photoelectric detection circuit and a processor, and an optical detection device provided with a detection zone and a blank zone for measuring assay results, and the photoelectric detection circuit detects the light-reflection intensity signal, and feeds back the detected information to the processor. The processor includes a time controlling module, a threshold modification module and a datum comparison module which are connected to each other, the time controlling module is connected with the threshold modification module and the photoelectric detection circuit separately, and the threshold modification module and the photoelectric detection circuit are connected with the datum comparison module at the same time, and the threshold modification module modifies threshold value according to the signal of time controlling module and sends to the datum comparison module, and the photoelectric detection circuit measures obtained detection value according to the signal of time controlling module and sends it to the datum comparison module, and the datum comparison module obtains the determined results by calculating and comparing detection data with corresponding the threshold value.

The determined results are outputted by a displaying device connected with the processor, and the displaying device can display the measuring status, and in this embodiment, the displaying device is a LCD liquid crystal screen. As a preferred embodiment, the LCD liquid crystal screen shows the detection status with an hourglass lightning picture.

The device for determining the result of assay further includes a voice alarming device connected with the processor. The determined result or detection status can be presented in a way of sound. For example, within a certain time after adding sample, the voice alarming device alarms to warn that the measuring program starts, or alarms when the result is outputted.

Referring to Fig. 2, the threshold modification module includes a starting-up testing module, a time measuring module, a threshold updating module and a threshold output module which are connected with each other. The threshold modification module detects the starting state (i.e. the circuit is switched on) according to the starting-up test module, the time test module detects the time signal from the timing control module, and based on the time signal, the threshold value update module updates the new threshold value according to a preset relation of the threshold value variation with time, and outputs the updated threshold value to the datum comparison module through threshold output module.

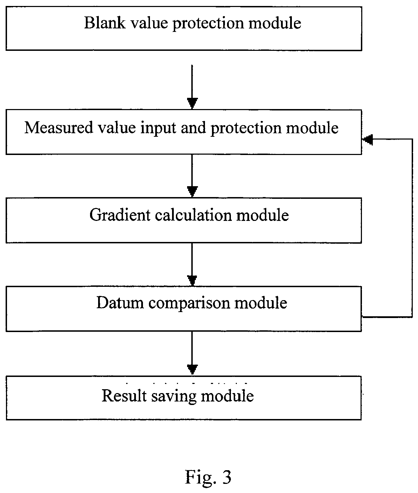

Referring to Fig. 3, the datum comparison module includes a blank value protection module, a measured value input and protection module, a gradient calculation module, a comparison and determination module, and a result saving module. Values of the blank value protection module, measured value input and protection module are inputted into gradient calculation module, and the results of the gradient calculation module are inputted into the comparison and determination module and compared with threshold values. The determined results are stored into the result saving module.

The device is used for reading result of assay to measure the existence and content of a target analyte, wherein the existence or non-existence of the target analyte gives a rise to a reaction and cause a signal accumulation with the time changing. The device is used to determine the accumulated signal cumulant, and calculate the determined value and compare it with the threshold value, and display the result of assay. Therefore, the device can guarantee the fast speed of obtaining the result and meanwhile the accurate result of test. It avoids the disadvantage that display error may happen with the short time after adding sample or that the waiting time is too long. Meanwhile, the most accurate result of the test is obtained at the fastest speed by fully considering the water loss of test strip and setting a relocatable threshold value. The setting of blank zone can remove the background, and reduce measuring errors.

The device can guarantee the fast speed of obtaining the result and meanwhile the accurate result of test. It avoids the disadvantage that display error may happen within a short time after adding sample or that the waiting time is too long. Meanwhile, the most accurate result of the test is obtained at the fastest speed by fully considering the water loss of test strip and setting a relocatable threshold value. The set of blank zone can remove the background, and reduce measuring errors.

In this embodiment, the assay is a cross flow assay, in which sample which may contain a special analyte is added to a liquid transport device (usually porous water-permeable carrier), and migrates along the device.

In the present invention, a method for determining the result of assay utilizing the device for determining the result of assay in the present invention is further provided. The method is used to measure the existence and content of an analyte to be determined, perform the assay to give rise to a reaction; the reaction causes an assay signal which accumulates with time in the detection zone of the optical detection device, and the photoelectric detection circuit emits light to the detection zone and the blank zone, and measures the light-reflection intensity signal of the detection zone and the blank zone, and feeds back detected information to the processor, wherein,
the processor is preset with a threshold value X changing with time;
the processor defines accumulated amount of the assay signal and calculates corresponding determined value K, according to the light-reflection intensity signals of both before and after adding sample of the detection zone and the blank zone, which are fed back by the photoelectric detection circuit;
when the confirmed determined value K is bigger than or equal to the current threshold value X, or at the time t, reaches the preset time value, the determined value K doesn't not reach the threshold value, or detected signal can not be determined, display the result.

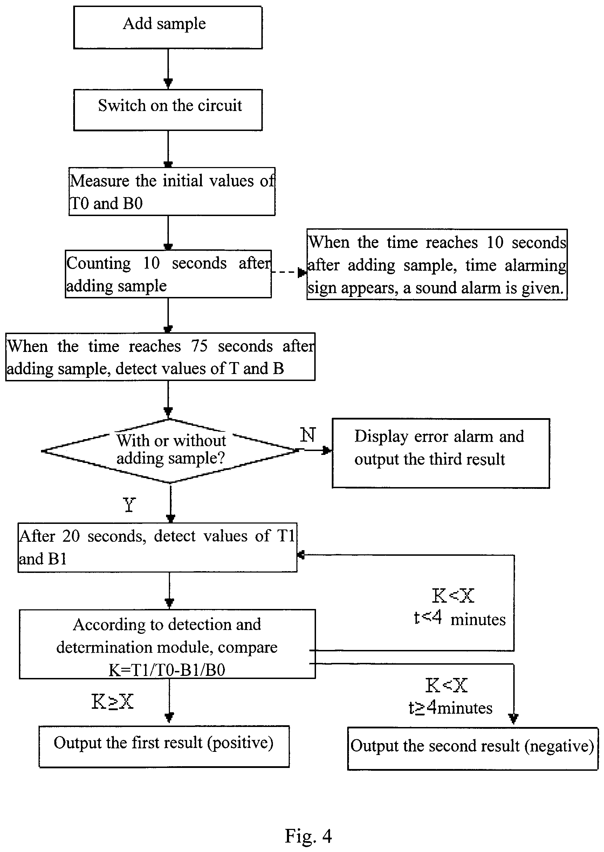

An embodiment is shown in Fig. 4, the photoelectricity circuit is switched on after adding sample, and measure and record the initial values of T0 and B0 of the detection zone and the blank zone, when time t reaches 10 second after adding sample, time alarming sign appears, and use a sound alarm to indicate that the sampling reaction starts.

When time t reaches 75 second after adding sample, reads values of T and B of the detection zone and blank zone, and determines whether sample is added, if no sample is added, which is T=T0 and B=B0, display error alarm and output the third result in a outputting way of displaying, and/or sound alarm; if a sample is added, T and B are different from T0 and B0, then move to the next step.

After a 20 second interval, read values of T1 and B1 of the detection zone and blank zone, with which the determined value K is calculated (K=T1/T0-B1/B0), and compared with threshold value X. If K≥X, output the first result, a positive result.

If within a preset time of 4 min, if the determined value K is smaller than preset threshold X (i.e. K<X), then repeat a new around of operation, i.e. reads the values of T1 and B1 every 20 second interval, recalculates the determined K and compares it with threshold X.

If the time t≥4 minutes, and the determined value K is still lower than the threshold value X, then output the second result, a negative result.

The accumulated signal in the assay process can be any signal suitable for above purpose. For convenience, the signal cumulant includes the formation or accumulation of the subjects easy for detection (for example the result of color reaction). More specifically, the assay better includes accumulation of markers, usually the accumulation of markers in the detection zone. The markers can be enzyme, radio-active isotope tracing, fluorescein, colloidal gold, colorful cream of latex, color particles and etc. Especially, the assay scheme utilizes functional micro-spheres of colloidal gold as markers.

Usually, a special analyte to be determined in the sample will cause accumulation of signal, but under some circumstances, like a competition reaction or the like, the existence of the special analyte to be determined will not cause relative signal accumulation.

Usually, within a certain time, after reaction, the determined value is bigger than or equal to preset threshold value, then immediately output the first result signal, the positive signal and end the assay.

If the determined value is smaller than preset threshold value within a certain time, then output the second result signal, the negative signal and end the assay.

If the sampling amount can not reach the detection limit after adding sample, then display unable to judge, and give a long alarm of beep.

The determined value is determined by that, scan and read values before sampling in the detection zone and the blank zone, and compare individually with the values which are scanned and read in the detection zone and the blank zone, and the ratio is obtained by subtracting the ratio of blank zone from the ratio of detection zone.

Therefore, the device can guarantee the fast speed of obtaining the result and meanwhile the accurate result of test. It avoids the disadvantage that error happens within a short time after adding sample or that the waiting time is too long. Meanwhile, the most accurate result of the test is obtained at the fastest speed by fully considering the water loss of test strip and setting a relocatable threshold value. The set of blank zone can remove the background, and reduce measuring errors.

The reaction causing the signal accumulation can be any suitable reaction, for example, a normal chemical reaction with two chemical substances, enzyme-linked immunosorbent assay, or immunity coupling reaction. The preferred immunity coupling reaction contains at least one biological molecule binding.

Preferred reactions contain combination between marker complex and special reagent fixed on the test strip of the detection zone, and markers accumulate in the combination zone.

After a liquid-absorbing stick is inserted into the sample liquid, while sample seeps to the liquid-absorbing stick, the electric resistance of circuit changes, the reading device is activated, and meanwhile scans the initial signal values of the detection zone and the blank zone. The whole process is completed automatically, but it may be accomplished with assistance of an extra switch or button.

After the reading device is activated, preset a certain time allowing the target subject completely react with marker reagent of the test strip, and displaying screen shows an hourglass picture. After a preset time interval (like 16 seconds), scan the values of detection zone and blank zone the second time, calculate determined value based on designed calculation formula, and compare the relation between determined value and threshold. If the determined value is bigger than the present threshold value, then, stops the assay safely and outputs positive result; if the determined value is less than present threshold, then restart a new around of measuring, calculating and comparing and the threshold is replaced by newly set threshold value; if the determined value is bigger than present threshold value, then, stops the assay safely and outputs positive result, if the determined value is smaller than present threshold value, then restart a new circle after the preset time. If within the preset time (i.e. the finishing point t), the determined value is still less than present threshold value, and the judgment is a negative result.

Typically, at the finishing point t, the device makes a conclusion that the assay ends, but t point is not definitely the end point of reaction.

In the aspect of finishing point t, special time point can be referred, i.e. calculate since sample liquid is added onto liquid-absorbing stick and the reading device is activated, preferably, t is chosen as 1-6 min.

The program in the processor can repeat the measurement for obtaining the final result. In a simple embodiment, the measurement will be repeated. Repeat once or many times before the finishing point, till the determined value exceeds the preset threshold value.

The device disclosed in present invention better comprises a clock or other timing devices, which makes sure automatic measurement at the preset time and no input or operation by the user is required.

In this way, measure at the initial time, if necessary, repeat the measurement at any expected time interval, till the determined value exceeds the preset threshold value.

The clock is used to preset interval time and calculate the finishing time to make the test go smoothly.

The device will measure one or multiple values and compare them based on preset program.

The above description is the detail and specific explanation for embodiments of the present invention, but it can not be understood as the restrictions on the scope of the present invention.

## Claims

1. A device for assay determination, comprising:
a photoelectric detection circuit;
a processor; and
an optical detection device for measuring assay signals, provided with a detection zone and a blank zone, wherein the photoelectric detection circuit detects light-reflection intensity signals from the detection zone and the blank zone, and feeds back the detected information to the processor;
wherein
the processor comprises a time controlling module, a threshold modification module and a datum comparison module which are connected to each other, the time controlling module being connected with the threshold modification module and the photoelectric detection circuit separately, and the threshold modification module and the photoelectric detection circuit being connected with the datum comparison module at the same time;
the threshold modification module modifies a threshold value according to signals from the time controlling module and a preset relation of the threshold value variation with time, and the modified threshold value is then sent to the datum comparison module;
the photoelectric detection circuit measures a detection value according to the signals from the time controlling module which is then sent to the datum comparison module; and
the datum comparison module obtains a determined assay result by calculating the detection value and comparing the same with the corresponding threshold value.

2. The device of claim 1, wherein the threshold modification module comprises a starting-up testing module, a time measuring module, a threshold updating module, and a threshold output module, which are connected with each other.

3. The device of claim 2, wherein the datum comparison module comprises a blank value protection module, a measured value input and protection module, a gradient calculation module, a comparison and determination module, and a result saving module, wherein:
values from the blank value protection module and the measured value input and protection module are inputted into the gradient calculation module;
the results from the gradient calculation module are inputted into the comparison and determination module and compared with the threshold value; and
the determined results are stored into the result saving module.

4. The device of claim 3, further comprising a displaying device for displaying the measuring status or determined result, wherein the displaying device is connected with the processor.

5. The device of claim 4, wherein the displaying device is a LCD liquid crystal display screen.

6. The device of claim 3, further comprising: a voice alarming device connected with the processor.

7. A method for assay determination by using the device according to any one of claims 1 to 6 to measure existence and content of an analyte to be determined, comprising:
performing an assay to give rise to a reaction that causes an assay signal which accumulates with time in the detection zone of the optical detection device;
the photoelectric detection circuit emitting light to the detection zone and the blank zone;
measuring light reflection intensity signals from the detection zone and the blank zone; and
feeding back the measured signals to the processor;
wherein:
the processor is preset with a threshold value X changing with time;
the processor determines the accumulated amount of the assay signal and calculates a corresponding determined value K, according to the light-reflection intensity signals of the detection zone and the blank zone both before and after adding a sample, which are fed back by the photoelectric detection circuit; and
one of the following assay results is determined and displayed:
the determined value K is bigger than or equal to the current threshold value X according to the preset relation of the threshold value variation with time; or
the determined value K doesn't not reach the threshold value when time t reaches a preset time value; or
the detected signal cannot be determined.

8. The method of claim 7, wherein:
if within the preset time value of time t after the reaction, the determined value is bigger than or equal to the preset threshold value, then immediately output a first result signal and end the assay;
if when the time t reaches the preset time value, the determined value is smaller than preset threshold value, then output a second result signal and end the assay; and
if no comparison status appears, output a third result signal and end the assay.

9. The method of claim 8, wherein the processor determines the accumulated amount of assay signal before the reaction reaches equilibrium.

10. The method of claim 9, wherein the determined value K is determined by the following steps:
reading a light reflection intensity signal from the detection zone as T0 and a light reflection intensity signal from the blank zone as B0 before adding a sample to the detection zone and the blank zone;
reading a light reflection intensity signal from the detection zone as T1 and a light reflection intensity signal from the blank zone as B1 after a time period after the sample is added to the detection zone and the blank zone;
calculating the ratio of T1/T0 for the detection zone and ratio of B1/B0 for the blank zone; and
calculating the determined value K by subtracting the ratio B1/B0 of the blank zone from the ratio T1/T0 of the detection zone.

11. The method of claim 8, wherein the first result is a positive result, the second result is a negative result, and the third result is a result of being unable to judge or insufficient sample.

12. The method of claim 8, wherein within the preset time value, if the determined value K is lower than the preset threshold value X, then repeat the steps in Claim 7.

## Patentansprüche

1. Vorrichtung für eine Versuchsbestimmung, Folgendes umfassend:
eine fotoelektrische Erfassungsschaltung;
einen Prozessor; und
eine optische Erfassungsvorrichtung zum Messen von Versuchssignalen, die mit einem Erfassungsbereich und einem Blindbereich versehen ist, wobei die fotoelektrische Erfassungsschaltung Lichtreflexionsintensitätssignale aus dem Erfassungsbereich und dem Blindbereich erfasst und die erfassten Informationen an den Prozessor zurückführt;
wobei der Prozessor ein Zeitsteuerungsmodul, ein Schwellenmodifikationsmodul und ein Bezugsgrößenvergleichsmodul umfasst, die miteinander verbunden sind, wobei das Zeitsteuerungsmodul separat mit dem Schwellenmodifikationsmodul und der fotoelektrischen Erfassungsschaltung verbunden ist und das Schwellenmodifikationsmodul und die fotoelektrische Erfassungsschaltung gleichzeitig mit dem Bezugsgrößenvergleichsmodul verbunden sind;
das Schwellenmodifikationsmodul einen Schwellenwert gemäß Signalen aus dem Zeitsteuerungsmodul und einer voreingestellten Beziehung der Schwellenwertvariation mit der Zeit modifiziert und der modifizierte Schwellenwert dann an das Bezugsgrößenvergleichsmodul gesendet wird;
die fotoelektrische Erfassungsschaltung einen Erfassungswert gemäß den Signalen aus dem Zeitsteuerungsmodul misst, der dann an das Bezugsgrößenvergleichsmodul gesendet wird; und
das Bezugsgrößenvergleichsmodul durch Berechnen des Erfassungswerts und Vergleichen desselben mit dem entsprechenden Schwellenwert ein bestimmtes Versuchsergebnis erhält.

2. Vorrichtung nach Anspruch 1, wobei das Schwellenmodifikationsmodul ein Anlauftestmodul, ein Zeitmessungsmodul, ein Schwellenaktualisierungsmodul und ein Schwellenausgabemodul umfasst, die miteinander verbunden sind.

3. Vorrichtung nach Anspruch 2, wobei das Bezugsgrößenvergleichsmodul ein Blindwertschutzmodul, ein Messwerteingabe- und Schutzmodul, ein Gradientenberechnungsmodul, ein Vergleichs- und Bestimmungsmodul und ein Ergebnisspeichermodul umfasst, wobei:
Werte aus dem Blindwertschutzmodul und dem Messwerteingabe- und Schutzmodul in das Gradientenberechnungsmodul eingegeben werden;
die Ergebnisse aus dem Gradientenberechnungsmodul in das Vergleichs- und Bestimmungsmodul eingegeben und mit dem Schwellenwert verglichen werden; und
die bestimmten Ergebnisse in dem Ergebnisspeichermodul gespeichert werden.

4. Vorrichtung nach Anspruch 3, ferner umfassend eine Anzeigevorrichtung zum Anzeigen des Messungszustands oder bestimmten Ergebnisses, wobei die Anzeigevorrichtung mit dem Prozessor verbunden ist.

5. Vorrichtung nach Anspruch 4, wobei die Anzeigevorrichtung ein LCD-Flüssigkristallanzeigebildschirm ist.

6. Vorrichtung nach Anspruch 3, ferner Folgendes umfassend:
eine Sprachalarmierungsvorrichtung, die mit dem Prozessor verbunden ist.

7. Verfahren für eine Versuchsbestimmung durch Verwenden der Vorrichtung nach einem der Ansprüche 1 bis 6, um ein Vorhandensein und einen Inhalt eines Analyten, der bestimmt werden soll, zu messen, Folgendes umfassend:
Durchführen eines Versuchs, um eine Reaktion zu verursachen, die ein Versuchssignal auslöst, das sich mit der Zeit in dem Erfassungsbereich der optischen Erfassungsvorrichtung ansammelt;
Ausstrahlen von Licht der fotoelektrischen Erfassungsschaltung zu dem Erfassungsbereich und dem Blindbereich;
Messen von Lichtreflexionsintensitätssignalen aus dem Erfassungsbereich und dem Blindbereich; und
Zurückführen der gemessenen Signale an den Prozessor;
wobei:
der Prozessor mit einem Schwellenwert X voreingestellt ist, der sich mit der Zeit ändert;
der Prozessor die angesammelte Menge des Versuchssignals bestimmt und einen entsprechenden bestimmten Wert K gemäß den Lichtreflexionsintensitätssignalen des Erfassungsbereichs und des Blindbereichs sowohl vor als auch nach dem Hinzufügen einer Probe berechnet, die durch die fotoelektrische Erfassungsschaltung zurückgeführt werden; und
eines der folgenden Versuchsergebnisse bestimmt und angezeigt wird:
der bestimmte Wert K ist größer als oder gleich dem aktuellen Schwellenwert X gemäß der voreingestellten Beziehung der Schwellenwertvariation mit der Zeit; oder
der ermittelte Wert K erreicht nicht den Schwellenwert, wenn die Zeit t einen voreingestellten Zeitwert erreicht; oder
das erfasste Signal kann nicht bestimmt werden.

8. Verfahren nach Anspruch 7, wobei:
sofern innerhalb des voreingestellten Zeitwerts der Zeit t nach der Reaktion der bestimmte Wert größer als oder gleich dem voreingestellten Schwellenwert ist, dann ein erstes Ergebnissignal sofort ausgegeben wird und der Versuch beendet wird;
sofern, wenn die Zeit t den voreingestellten Zeitwert erreicht, der ermittelte Wert kleiner als der voreingestellte Schwellenwertwert ist, dann ein zweites Ergebnissignal ausgegeben wird und der Versuch beendet wird; und
sofern kein Vergleichszustand erscheint, ein drittes Ergebnissignal ausgegeben wird und der Versuch beendet wird.

9. Verfahren nach Anspruch 8, wobei der Prozessor die angesammelte Menge des Versuchssignals bestimmt, bevor die Reaktion ein Gleichgewicht erreicht.

10. Verfahren nach Anspruch 9, wobei der bestimmte Wert K durch die folgenden Schritte bestimmt wird:
Lesen eines Lichtreflexionsintensitätssignals aus dem Erfassungsbereich als T0 und eines Lichtreflexionsintensitätssignals aus dem Blindbereich als B0, bevor eine Probe zu dem Erfassungsbereich und dem Blindbereich hinzugefügt wird;
Lesen eines Lichtreflexionsintensitätssignals aus dem Erfassungsbereich als T1 und eines Lichtreflexionsintensitätssignals aus dem Blindbereich als B1 nach einer Zeitspanne, nachdem die Probe zu dem Erfassungsbereich und dem Blindbereich hinzugefügt wurde;
Berechnen des Verhältnisses von T1/T0 für den Erfassungsbereich und des Verhältnisses von B1/B0 für den Blindbereich; und
Berechnen des bestimmten Werts K durch Subtrahieren des Verhältnisses B1/B0 des Blindbereichs von dem Verhältnis T1/T0 des Erfassungsbereichs.

11. Verfahren nach Anspruch 8, wobei das erste Ergebnis ein positives Ergebnis ist, das zweite Ergebnis ein negatives Ergebnis ist und das dritte Ergebnis ein Ergebnis eines Außerstandeseins des Beurteilens oder einer unzureichenden Probe ist.

12. Verfahren nach Anspruch 8, wobei innerhalb des voreingestellten Zeitwerts, sofern der bestimmte Wert K niedriger als der voreingestellte Schwellenwertwert X ist, dann die Schritte in Anspruch 7 wiederholt werden.

## Revendications

1. Dispositif de détermination de dosage, comprenant :
un circuit de détection photoélectrique ;
un processeur ; et
un dispositif de détection optique pour mesurer des signaux de dosage, pourvu d'une zone de détection et d'une zone vierge, dans lequel le circuit de détection photoélectrique détecte des signaux d'intensité de réflexion de lumière de la zone de détection et de la zone vierge et renvoie les informations détectées au processeur ;
dans lequel
le processeur comprend un module de commande de temps, un module de modification de seuil et un module de comparaison de données connectés l'un à l'autre, le module de commande de temps étant connecté au module de modification de seuil et au circuit de détection photoélectrique séparément, et le module de modification de seuil et le circuit de détection photoélectrique étant connectés au module de comparaison de données en même temps ;
le module de modification de seuil modifiant une valeur de seuil en fonction de signaux du module de commande de temps et d'une relation prédéfinie de la variation de la valeur de seuil avec le temps, la valeur de seuil modifiée étant ensuite envoyée au module de comparaison de données ;
le circuit de détection photoélectrique mesurant une valeur de détection en fonction des signaux du module de commande de temps qui est ensuite envoyée au module de comparaison de données ; et
le module de comparaison de données obtenant un résultat de dosage déterminé en calculant la valeur de détection et en la comparant à la valeur de seuil correspondante.

2. Dispositif selon la revendication 1, dans lequel le module de modification de seuil comprend un module de test de démarrage, un module de mesure de temps, un module de mise à jour de seuil et un module de sortie de seuil, qui sont connectés l'un à l'autre.

3. Dispositif selon la revendication 2, dans lequel le module de comparaison de données comprend un module de protection de valeur vierge, un module d'entrée de valeur mesurée et de protection, un module de calcul de gradient, un module de comparaison et de détermination, et un module de sauvegarde de résultat, dans lequel :
des valeurs du module de protection de valeur vierge et du module d'entrée de valeur mesurée et de protection sont entrées dans le module de calcul de gradient ;
les résultats du module de calcul de gradient sont entrés dans le module de comparaison et de détermination et comparés à la valeur de seuil ; et
les résultats déterminés sont stockés dans le module de sauvegarde de résultat.

4. Dispositif selon la revendication 3, comprenant en outre un dispositif d'affichage pour afficher l'état de mesure ou un résultat déterminé, dans lequel le dispositif d'affichage est connecté au processeur.

5. Dispositif selon la revendication 4, dans lequel le dispositif d'affichage est un écran d'affichage à cristaux liquides LCD.

6. Dispositif selon la revendication 3, comprenant en outre : un dispositif d'alarme vocale connecté au processeur.

7. Procédé de détermination de dosage à l'aide du dispositif selon l'une quelconque des revendications 1 à 6 pour mesurer l'existence et le contenu d'un analyte à déterminer, consistant à :
effectuer un dosage pour donner lieu à une réaction qui provoque un signal de dosage qui s'accumule dans le temps dans la zone de détection du dispositif de détection optique ;
le circuit de détection photoélectrique émettant de la lumière vers la zone de détection et la zone vierge ;
mesurer des signaux d'intensité de réflexion de lumière de la zone de détection et de la zone vierge ; et
renvoyer les signaux mesurés au processeur ;
dans lequel :
le processeur est prédéfini avec une valeur de seuil X évoluant dans le temps ;
le processeur détermine la quantité accumulée du signal de dosage et calcule une valeur déterminée correspondante K, en fonction des signaux d'intensité de réflexion de lumière de la zone de détection et de la zone vierge à la fois avant et après l'ajout d'un échantillon, qui sont renvoyés par le circuit de détection photoélectrique ; et
l'un des résultats de dosage suivants est déterminé et affiché :
la valeur déterminée K est supérieure ou égale à la valeur de seuil actuelle X en fonction de la relation prédéfinie de la variation de valeur de seuil dans le temps ; ou
la valeur déterminée K n'atteint pas la valeur de seuil lorsque le temps t atteint une valeur temporelle prédéfinie ; ou
le signal détecté ne peut pas être déterminé.

8. Procédé selon la revendication 7, dans lequel :
si, à l'intérieur de la valeur temporelle prédéfinie du temps t après la réaction, la valeur déterminée est supérieure ou égale à la valeur de seuil prédéfinie, alors immédiatement un premier signal de résultat est émis et le dosage est terminé ;
si, lorsque le temps t atteint la valeur temporelle prédéfinie, la valeur déterminée est inférieure à la valeur de seuil prédéfinie, alors un deuxième signal de résultat est émis et le dosage est terminé ; et
si aucun état de comparaison n'apparaît, un troisième signal de résultat est émis et le dosage est terminé.

9. Procédé selon la revendication 8, dans lequel le processeur détermine la quantité accumulée de signal de dosage avant que la réaction n'atteigne un équilibre.

10. Procédé selon la revendication 9, dans lequel la valeur déterminée K est déterminée par les étapes suivantes consistant à :
lire un signal d'intensité de réflexion de lumière de la zone de détection en tant que T0 et un signal d'intensité de réflexion de lumière de la zone vierge en tant que B0 avant d'ajouter un échantillon à la zone de détection et à la zone vierge ;
lire un signal d'intensité de réflexion de lumière de la zone de détection en tant que T1 et un signal d'intensité de réflexion de lumière de la zone vierge en tant que B1 après une période de temps après l'ajout de l'échantillon à la zone de détection et à la zone vierge ;
calculer le rapport de T1/T0 pour la zone de détection et le rapport de B1/B0 pour la zone vierge ; et
calculer la valeur déterminée K par soustraction du rapport B1/B0 de la zone vierge du rapport T1/T0 de la zone de détection.

11. Procédé selon la revendication 8, dans lequel le premier résultat est un résultat positif, le deuxième résultat est un résultat négatif et le troisième résultat est un résultat d'incapacité à juger ou d'un échantillon insuffisant.

12. Procédé selon la revendication 8, dans lequel, à l'intérieur de la valeur temporelle prédéfinie, si la valeur déterminée K est inférieure à la valeur de seuil prédéfinie X, répéter alors les étapes selon la revendication 7.
